(19) Europäisches Patentamt
European Patent Office
Office européen des brevets

(11) **EP 4 018 951 A1**

(12) **EUROPEAN PATENT APPLICATION**

(43) Date of publication:
**29.06.2022 Bulletin 2022/26**

(21) Application number: **21217671.3**

(22) Date of filing: **24.12.2021**

(51) International Patent Classification (IPC):
***A61B 18/12*** (2006.01)   ***A61B 18/14*** (2006.01)
***A61B 18/00*** (2006.01)

(52) Cooperative Patent Classification (CPC):
**A61B 18/1233; A61B 18/1492;** A61B 2018/0022;
A61B 2018/00357; A61B 2018/00577;
A61B 2018/00642; A61B 2018/00702;
A61B 2018/0075; A61B 2018/00767;
A61B 2018/00869; A61B 2018/00892

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.12.2020 US 202017135236**

(71) Applicant: **Biosense Webster (Israel) Ltd.
Yokneam, 2066717 (IL)**

(72) Inventors:
• **GOVARI, Assaf
Yokneam 2066717 (IL)**
• **ALTMANN, Andres Claudio
Yokneam 2066717 (IL)**

(74) Representative: **Carpmaels & Ransford LLP
One Southampton Row
London WC1B 5HA (GB)**

(54) **CONTROLLING INTER-ELECTRODE CURRENTS DURING ABLATION**

(57)   A medical apparatus includes a probe, which includes an insertion tube configured for insertion into a body cavity of a patient, and a distal assembly, which is connected distally to the insertion tube and includes a plurality of electrodes, which are configured to contact tissue within the body cavity. An electrical signal generator is configured to apply radio frequency (RF) signals simultaneously to the plurality of electrodes with energy sufficient to ablate the tissue contacted by the electrodes. A controller is coupled to measure time-varying voltage differences between the electrodes and to adjust the RF signals applied to the electrodes responsively to the measured time-varying voltage differences.

FIG. 2

**Description**

**FIELD OF THE INVENTION**

The present invention relates generally to medical devices, and particularly to devices and methods for ablation of physiological tissues.

**BACKGROUND**

Radio frequency ablation (RFA) is a medical procedure in which part of the electrical conduction pathways of the heart or other dysfunctional tissue are ablated using the heat generated from radio frequency alternating current (for example in the frequency range of 350-500 kHz). The ablation is done by inserting a probe, such as a catheter, into the tissue, and applying the RF current to electrodes at the tip of the probe.

United States Patent 6,584,345 describes an apparatus for measuring electrical signals emanating from a body of a patient, and, in particular, from the patient's heart. The apparatus includes a catheter having an electrode array, preferably on its distal end. The apparatus of the invention further includes a first amplifier for measuring a voltage from a first electrode of the array, and a cascade of differential amplifiers, each of which measures a voltage difference between two successive electrodes in the array.

United States Patent 10,182,742 describes a method and system for assessing electrode-tissue contact before the delivery of ablation energy. The system may include a control unit programmed to determine a difference between a maximum impedance magnitude at a low frequency for a given electrode and an absolute minimum impedance magnitude at the low frequency across all electrodes, determine a difference between a maximum impedance magnitude at a high frequency for a given electrode and an absolute minimum impedance magnitude at the high frequency across all electrodes, and determine a difference between a maximum impedance phase at the high frequency for a given electrode and an absolute minimum impedance phase at the high frequency across all electrodes.

United States Patent Application Publication 2018/0042674 describes catheter systems and methods for the selective and rapid application of DC voltage to drive irreversible electroporation. In some embodiments, an apparatus includes a voltage pulse generator and an electrode controller. The voltage pulse generator is configured to produce a pulsed voltage waveform. The electrode controller is operably coupled to the voltage pulse generator and a medical device including a series of electrodes. The electrode controller includes a selection module and a pulse delivery module. The selection module selects a subset of electrodes from the series of electrodes and identifies at least one electrode as an anode and at least one electrode as a cathode. The pulse delivery module delivers an output signal associated with the pulsed voltage waveform to the subset of electrodes.

**SUMMARY**

Embodiments of the present invention that are described hereinbelow provide improved apparatus and methods for ablation of body tissues.

There is therefore provided, in accordance with an embodiment of the invention, medical apparatus, including a probe, which includes an insertion tube configured for insertion into a body cavity of a patient, and a distal assembly, which is connected distally to the insertion tube and includes a plurality of electrodes, which are configured to contact tissue within the body cavity. An electrical signal generator is configured to apply radio frequency (RF) signals simultaneously to the plurality of electrodes with energy sufficient to ablate the tissue contacted by the electrodes. A controller is coupled to measure time-varying voltage differences between the electrodes and to adjust the RF signals applied to the electrodes responsively to the measured time-varying voltage differences.

In some embodiments, the controller is configured to adjust the RF signals applied to the electrodes so that the voltage differences do not exceed a predetermined threshold at any time during application of the RF signals. In one embodiment, the controller is configured to adjust an amplitude of the RF signals so as to compensate for a difference in respective peak voltages measured at a given pair of the electrodes. Additionally or alternatively, the controller is configured to adjust a phase of the RF signals so as to compensate for a phase offset between respective voltage waveforms measured at a given pair of the electrodes.

In a disclosed embodiment, the distal assembly includes a balloon, which is connected distally to the insertion tube and is configured to be inflated within the body cavity with a fluid that flows into the balloon through the insertion tube.

In some embodiments, the apparatus includes a common electrode configured to be fixed to a location on the body of the patient, so that the RF signals pass through the body from the plurality of the electrodes on the probe to the common electrode, thereby ablating the tissue in a unipolar mode. In a disclosed embodiment, the controller is configured to adjust the RF signals applied to the electrodes so as to control a proportionality between a unipolar RF current flowing from the electrodes on the probe to the common electrode and a bipolar RF current flowing between two or more of the electrodes on the probe, thereby ablating the tissue simultaneously in the unipolar mode and in a bipolar mode. In one embodiment, the controller is configured to control the proportionality between the unipolar and bipolar RF currents by adjusting a phase offset between respective voltage waveforms applied to the two or more of the electrodes on the probe.

There is also provided, in accordance with an embodiment of the invention, a method for medical treatment and diagnostics. The method includes providing a probe for insertion into a body cavity of a patient, wherein the probe includes an insertion tube and a distal assembly, which is connected distally to the insertion tube and includes a plurality of electrodes, which are configured to contact tissue within the body cavity. Radio-frequency (RF) signals are applied simultaneously in parallel to the plurality of electrodes with an energy sufficient to ablate the tissue contacted by the electrodes. Time-varying voltage differences between the electrodes are measured, and the RF signals applied to the electrodes are adjusted responsively to the measured time-varying voltage differences.

The present invention will be more fully understood from the following detailed description of the embodiments thereof, taken together with the drawings in which:

## BRIEF DESCRIPTION OF THE DRAWINGS

Fig. 1 is a schematic pictorial illustration of a medical apparatus in the course of a unipolar RFA procedure, in accordance with an embodiment of the invention; and

Fig. 2 is a schematic circuit diagram of an electrical signal generator used in an RFA procedure, in accordance with an embodiment of the invention.

## DETAILED DESCRIPTION OF EMBODIMENTS

In a radio-frequency ablation (RFA) procedure, an alternating electrical current, typically with a frequency between 350 and 500 kHz, is driven through the tissue of a subject. The electrical current is carried into the tissue through the electrodes of a catheter inserted in the tissue.

Some RFA procedures use a balloon catheter, which has a balloon at its distal end and electrodes arrayed around the surface of the balloon. The balloon is inflated within the body cavity, and the electrodes are then brought into contact with the tissue that is to be ablated. For ablating tissue in small cavities within the body, for example in the left atrium of the heart, balloons of small diameter can be used, for example with a diameter less than 15 mm.

RFA may be carried out in either a bipolar mode, in which the ablation currents flow from one ablation electrode to another on the same catheter, or a unipolar mode, in which the ablation currents flow between the ablation electrodes on the catheter and an external electrode, or "return patch." The return patch is typically fixed to the body surface of the subject, for example on the skin of the subject's torso, with an electrical return connection to the RF signal generator.

For a number of reasons, including their small size, small-diameter balloons are commonly used in a unipolar ablation mode. However, due to the small size of the balloon, the electrodes on the surface of the balloon are close together, and even a small voltage difference between any two electrodes may cause stray currents to flow between the two electrodes, rather than from each electrode through the tissue to the return patch. This unintended change in the current paths may affect the outcome of the ablation procedure. Even when the amplitudes (peak voltages) of the RF signals on the electrodes are equal, the phases of the signals at the electrodes may be different due to differences in the electrical impedances of the respective current pathways, thus causing stray currents to flow between the electrodes.

Some of the embodiments of the present invention that are described herein address this problem by measuring and correcting for time-varying voltage differences between the electrodes on a probe during an RFA procedure. In these embodiments, an electrical signal generator applies RF signals simultaneously in parallel to multiple electrodes on the probe, with an energy sufficient to ablate the tissue contacted by the electrodes in a unipolar ablation procedure. A controller measures the time-varying voltage differences between the electrodes and adjusts the RF signals applied to the electrodes so that the voltage differences do not exceed a predetermined threshold at any time during the application of the RF signals. This approach ensures that any stray currents between pairs of electrodes remain sufficiently low so as not to affect the intended ablation of the RFA procedure.

Typically, the controller separately measures and adjusts the amplitudes and phases of the RF signals applied to the different electrodes so as to compensate for differences in respective peak voltages and/or phase offsets between the respective voltage waveforms measured at any given pair of the electrodes.

In other embodiments, the controller measures the time-varying voltage differences and adjusts the RF signals so that a well-defined voltage difference is created between one or more pairs of the electrodes. This voltage difference can be controlled, for example, by adjusting the relative phases between the RF signals that are applied to electrodes in each such pair. By appropriate setting of the time-varying voltage difference, the proportionality between the unipolar RF current flowing from the electrodes to the return patch and the bipolar RF current flowing between two or more of the electrodes can be precisely controlled. Using this approach, it is possible to perform unipolar and bipolar ablation simultaneously, and thus flexibly control the depth and extent of the ablation lesions.

SYSTEM DESCRIPTION

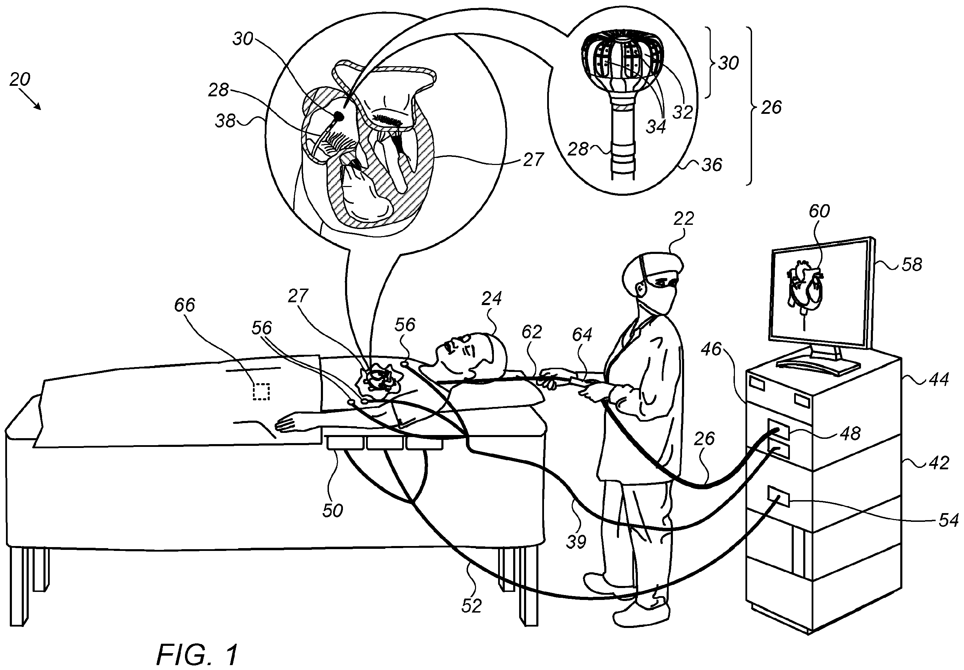

Fig. 1 is a schematic pictorial illustration of a medical apparatus 20 in the course of a unipolar RFA procedure, in accordance with an embodiment of the invention. A physician 22 performs the RFA procedure on a patient 24, using an ablation catheter 26, with further details of the catheter described hereinbelow. The embodiment shown in the figures refers to an example of an RFA procedure in a chamber of a heart 27 using a balloon 32. In alternative embodiments, the RFA procedure may be performed using other types of catheters having multiple electrodes and may be performed not only in heart 27, but also in other organs and tissue, as will be apparent to those skilled in the art after reading the present description.

As shown in an inset 36, ablation catheter 26 comprises a shaft 28 and a distal assembly 30, wherein the shaft functions as an insertion tube for inserting the distal assembly into a body cavity patient 24, in this case into the chamber of heart 27. Distal assembly 30 comprises balloon 32 with a plurality of ablation electrodes 34. Distal assembly 30 and a part of shaft 28 are also shown in an inset 38. In alternative embodiments, distal assembly 30 may comprise a structure different from a balloon.

Medical apparatus 20 further comprises a controller 42 and an electrical signal generator 44, typically residing in a console 46; the controller and the signal generator may each comprise one or several circuit components. Catheter 26 is connected to console 46 via an electrical interface 48, such as a port or socket, through which RF signals are carried from signal generator 44 to distal assembly 30.

Controller 42 receives from physician 22 (or another operator), prior to and/or during the ablation procedure, setup parameters for the procedure. For example, using one or more suitable input devices, such as a keyboard, mouse, or touch screen (not shown), physician 22 defines the electrical and temporal parameters of the RFA signals to be applied to selected segments of electrodes 34. Controller 42 passes suitable control signals to signal generator 44 for performing the RFA.

Controller 42 may be further configured to track the respective positions of electrodes 34 during the RFA procedure and during electrophysiological signal acquisition, using any suitable tracking technique. For example, distal assembly 30 may comprise one or more electromagnetic position sensors (not shown), which, in the presence of an external magnetic field generated by one or more magnetic-field generators 50, output signals that vary with the positions of the sensors. Based on these signals, controller 42 may ascertain the positions of electrodes 34. Magnetic-field generators 50 are connected to console 46 via cables 52 and an interface 54. Alternatively, for each electrode 34, controller 42 may ascertain the respective impedances between the electrode and multiple external electrodes 56 coupled to patient 24 at various different locations, and then compute the ratios between these impedances, these ratios being indicative of the electrode's location. As yet another alternative, the controller may use both electromagnetic tracking and impedance-based tracking, as described, for example, in US Patent 8,456,182, whose disclosure is incorporated herein by reference.

In some embodiments, controller 42 displays, on a display screen 58, a relevant image 60 of the subject's anatomy, annotated, for example, to show the current position and orientation of distal assembly 30.

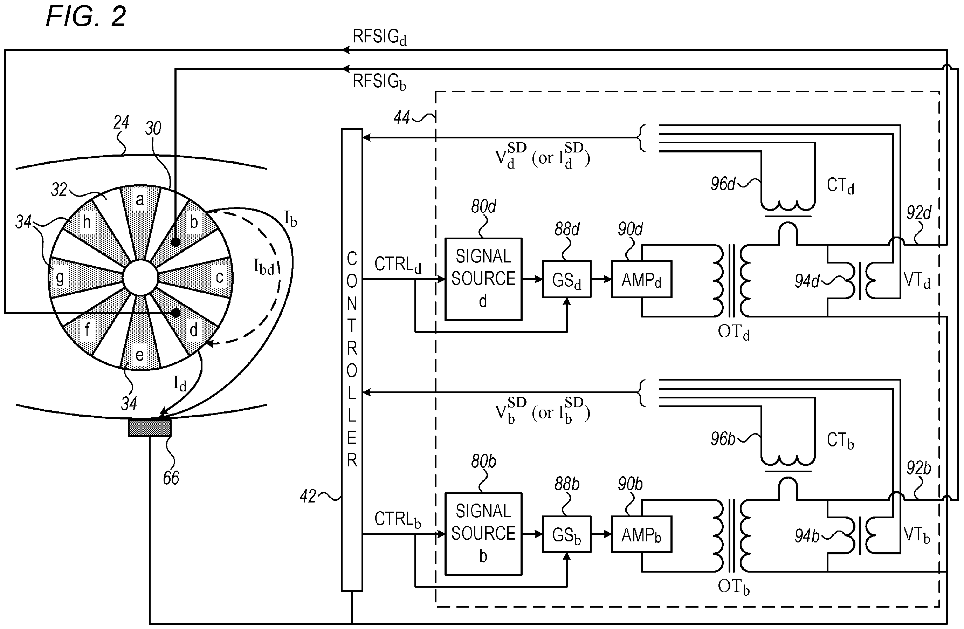

Controller 42 and electric signal generator 44 may typically comprise both analog and digital elements. Thus, controller 42 comprises an analog front-end with multiple inputs with respective analog-to-digital converters (ADCs) for monitoring the RF ablation signals applied by signal generator 44 to each of electrodes 34. Controller 42 further comprises multiple digital output circuits for sending commands to signal generator 44 for adjusting the RF signals, as detailed in Fig. 2 hereinbelow.

Electric signal generator 44 typically comprises RF analog circuits for generating and amplifying the RF signals for ablation, as well as digital input circuits for receiving digital control signals from controller 42.

Alternatively, the control signals may be passed from controller 42 to electric signal generator 44 in an analog form, provided that the controller and the signal generator are configured accordingly.

Typically, the functionality of controller 42, as described herein, is implemented at least partly in software. For example, controller 42 may comprise a programmed digital computing device comprising at least a central processing unit (CPU) and random access memory (RAM). Program code, including software programs, and/or data are loaded into the RAM for execution and processing by the CPU. The program code and/or data may be downloaded to the controller in electronic form, over a network, for example. Alternatively or additionally, the program code and/or data may be provided and/or stored on non-transitory tangible media, such as magnetic, optical, or electronic memory. Such program code and/or data, when provided to the controller, produce a machine or special-purpose computer, configured to perform the tasks described herein.

At the start of the RFA procedure, physician 22 inserts catheter 26 through a sheath 62 with balloon 32 in a collapsed configuration, and only after the catheter exits the sheath is the balloon inflated to its intended functional shape with a fluid that flows into the balloon through shaft 28. This functional shape is shown in insets 36 and 38. By containing balloon 32 in a collapsed configuration, sheath 62 also serves to minimize vascular trauma while the balloon is brought to the target location. Physician 22 navigates catheter 26 to a target location in heart 27 of patient 24, by manipulating the catheter, using a manipulator 64 near the proximal end of the catheter, and/or deflection from sheath 62. Physician 22 brings distal assembly 30 into contact with tissue, such as myocardial tissue, of heart 27. Next, under the control of physician 22 and controller 42, electrical signal generator 44 generates RFA signals, which are carried through catheter 26 over different respective channels, to ablation electrodes 34. A typical RF energy required for RFA exceeds 100 J.

In unipolar RFA, the currents of ablation signals flow between ablation electrodes 34 and an external electrode, or "return patch" 66, which is coupled externally between patient 24, typically on the skin of the subject's torso, and signal generator 44. However, if the RF voltages and phases of neighboring electrodes 34 are not equal, some of the RF currents will flow as stray currents between the electrodes, and the ablation of the tissue will not take place as intended by physician 22. In order to prevent these stray currents, controller 42 monitors the RF voltages applied by signal generator 44 to electrodes 34. As will be further detailed in Fig. 2 hereinbelow, controller 42 adjusts the RF signals applied to electrodes 34 so that the voltage differences do not exceed a predetermined threshold at any time during application of the RF signals.

Alternatively or additionally, controller 42 may adjust the RF signals so as to apply an intentional, well-controlled voltage difference between two or more of electrodes 34, for example by introducing a phase offset between the signals applied to the electrodes. In this case, both unipolar and bipolar currents will flow through the tissue of heart 27 in a proportionality determined by the voltage difference. For example, in one embodiment, controller 42 controls the proportionality between the unipolar and bipolar RF currents by adjusting a phase offset between the respective voltage waveforms that are applied to the two or more of electrodes 34.

Notwithstanding the particular type of ablation procedure illustrated in Fig. 1, it is noted that the embodiments described herein may be applied to any suitable type of multi-channel radio-frequency ablation procedure.

Fig. 2 is a schematic circuit diagram of electrical signal generator 44, in accordance with an embodiment of the invention. The same labels are used to indicate items identical or similar to the corresponding items in Fig. 1.

Distal assembly 30 is shown in Fig. 2 from a distal point of view along the axis of shaft 28. In the pictured embodiment, distal assembly 30 comprises balloon 32 and eight electrodes 34a-34h, and is positioned within heart 27 (Fig. 1) of patient 24 for the purpose of RFA. Connections and internal circuitry of signal generator 44 are shown in Fig. 2, for the sake of clarity, only for electrodes 34b and 34d rather than for all eight electrodes. Subscripts b and d are used in the figure to identify connections and circuit components specifically associated with respective electrodes 34b and 34d.

Signal generator 44 comprises multiple signal sources 80, which generate, in response to control signals CTRL received from controller 42, RF signals RFSIG for application to patient 24 through ablation electrodes 34. The signal path is closed through return patch 66. In the present embodiment, signal sources 80 are voltage sources, wherein the amplitude and phase of the output voltage of each signal source are set by external control signal CTRL. Alternatively, signal sources 80 may comprise current sources, wherein the amplitude and phase of the output current are set by the external control signal.

In general, signal generator 44 may comprise any suitable number of signal sources 80, corresponding to the number of ablation electrodes 34. For example, signal generator 44 may comprise from two to twenty signal sources 80. Each signal source 80 may comprise, for example, a digital signal source coupled to a digital-to-analog converter, a stable analog free-running generator, or a direct digital synthesizer (DDS), such as the AD9854 DDS made by Analog Devices, Inc. (Norwood, Massachusetts, USA). Each signal source 80 generates an analog output waveform with amplitude and phase adjusted in accordance with the corresponding control signal CTRL.

Signal generator 44 further comprises multiple controlled gain stages 88 (labelled as GS), configured to adjust the respective amplitudes of the composite signals during the application of the composite signals to the subject. Typically, signal generator 44 comprises one controlled gain stage 88 for each signal source 80. Each controlled gain stage 88 may comprise, for example, a digital potentiometer, such as the AD5122 digital potentiometer by Analog Devices.

Typically, signal generator 44 further comprises multiple amplifiers 90, configured to amplify the adjusted signals received from controlled gain stages 88. The amplified RF signals are output to ablation electrodes 44, over respective channels 92. Typically, output transformers OT provide electrical isolation between the electrodes and signal generator 44.

Signal generator 44 further comprises, for each channel 92, a voltage transformer 94 and/or a current transformer 96, configured to step-down the voltage and/or current of the respective RF signal to measurable levels, and with known step-down ratios. When signal sources 80 are configured as voltage sources, the stepped-down voltage for each channel 92 (e.g., the voltage induced across each voltage transformer 94, and indicated by $V^{SD}$) is input to the analog front-end of controller 42, and is then converted, by the analog-to-digital converter, to a digital signal. In an alternative embodiment, wherein signal sources 80 function as current sources, a stepped-down current $I^{SD}$ for each channel 92 is input to the analog front-end of controller 42, and converted, by the analog-to-digital converter, to a digital signal.

Each RF signal generates a corresponding voltage V(t) at the respective electrode 34. V(t) varies sinusoidally as a function of time t and may be written in the general form:

$$V(t) = A\sin(2\pi f t + \emptyset),$$

wherein A is the amplitude of the signal, f is the frequency of the signal (for example in the frequency range of 350-500 kHz), and $\Phi$ is the phase of the signal.

Following this notation, the RF voltages on electrodes 34b and 34d are specifically given by:

$$V_b(t) = A_b \sin(2\pi f t + \emptyset_b)$$

and

$$V_d(t) = A_d \sin(2\pi f t + \emptyset_d).$$

These RF voltages $V_b(t)$ and $V_d(t)$ drive respective ablation currents $I_b$ and $I_d$ from electrodes 34b and 34d through patient 24 to return patch 66. If the amplitudes and/or phases of the RF voltages on electrodes 34b and 34d differ from each other, a stray current $I_{bd}$ is driven between the two electrodes. This stray current may alter the effect of the RFA.

In order to minimize the potential stray current $I_{bd}$, controller 42 computes, using the received stepped-down voltages $V^{SD}_b$ and $V^{SD}_d$ and the known step-down ratios of transformers 94b and 94d, the actual voltages $V_b(t)$ and $V_d(t)$ on electrodes 34b and 34d. Controller 42 measures the difference $V_b(t)- V_d(t)$ between the signals by computing the amplitudes (peak voltages) $A_b$ and $A_d$ and phases $\Phi_b$ and $\Phi_d$ (against a fixed timing signal) of RF signals $V_b(t)$ and $V_d(t)$. These measurements yield an amplitude difference $A_b-A_d$ and a phase offset (phase difference) $\Phi_b-\Phi_d$. In case either or both of these differences exceed predetermined thresholds, controller 42 issues commands, shown by arrows $CTRL_b$ and $CTRL_d$, to respective signal sources 80b and 80d. In response to these commands, signal sources 80b and 80d adjust amplitudes and phases $A_b,\Phi_b$ and $A_d,\Phi_d$ so that the amplitude and phase differences return to values that are less than predetermined thresholds. This ensures that any stray current $I_{bd}$ between electrodes 34b and 34d remains sufficiently low, so as to not significantly alter the intended effect of the RFA.

Although the present example relates only to a single pair of electrodes 34b and 34d, in practice controller 42 measures the voltages of all the electrodes, using the techniques described above, and adjusts all of the amplitudes and phases as necessary to ensure that all of the electrode voltages are equal to within the predetermined threshold.

In one embodiment, controller 42 extracts the amplitudes $A_b$ and $A_d$ and phases $\Phi_b$ and $\Phi_d$ using a four-point method, wherein controller 42 samples the voltages $V^{SD}_b$ and $V^{SD}_d$ at four discrete times separated by a quarter of a period of the signal: $t_0$, $t_1=t_0+\tau/4$, $t_2=t_0+\tau/2$, and $t_3=t_0+3\tau/4$, wherein $t_0$ is an arbitrarily chosen but fixed point in time, and $\tau=1/f$ is the period of the RF signal. As previously described, controller converts the sampled stepped-down voltages to the actual voltages $V_b(t)$ and $V_d(t)$ on electrodes 34b and 34d, now written for $V_b(t)$ in terms of discrete values corresponding to the four sampling times as $V_b(t_0)$, $V_b(t_1)$, $V_b(t_2)$, $V_b(t_3)$, and for $V_d(t)$ as $V_d(t_0)$, $V_d(t_1)$, $V_d(t_2)$, $V_d(t_3)$. From these values controller 42 computes the amplitudes and phases of the RF signals $V_b(t)$ and $V_d(t)$ using the following formulae:

$$A_b = \tfrac{1}{2}\sqrt{\left(V_b(t_0) - V_b(t_2)\right)^2 + \left(V_b(t_1) - V_b(t_3)\right)^2},$$

$$\emptyset_b = arctan\frac{V_b(t_0)-V_b(t_2)}{V_b(t_1)-V_b(t_3)},$$

$$A_d = \tfrac{1}{2}\sqrt{\left(V_d(t_0) - V_d(t_2)\right)^2 + \left(V_d(t_1) - V_d(t_3)\right)^2},$$

and

$$\emptyset_d = arctan\frac{V_d(t_0)-V_d(t_2)}{V_d(t_1)-V_d(t_3)}.$$

As alternatives to the above-described four-point method, other methods such as three-point or six-point methods may be used, with three points over the period $\tau$ being the minimum to yield unique results.

In an alternative embodiment, as noted earlier, controller 42 does not necessarily attempt to minimize the amplitude difference $A_b$-$A_d$ and a phase offset (phase difference) $\Phi_b$-$\Phi_d$. Rather, controller 42 issues commands $CTRL_b$ and $CTRL_d$ to signal sources 80b and 80d to adjust amplitudes and phases $A_b,\Phi_b$ and $A_d,\Phi_d$ to so as to give rise to a current $I_{bd}$ of a desired amplitude between electrodes 34b and 34d. For example, controller 42 may set the amplitudes $A_b$ and $A_d$ to the same value while maintaining a certain phase offset $\Phi_b$-$\Phi_d$. The current $I_{bd}$ will then be proportional to the sine of the phase offset, and the proportionality between the unipolar and bipolar currents can be controlled simply by changing the phase offset.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

Aspects of the invention:

1. A method for medical treatment and diagnostics, the method comprising:

providing a probe for insertion into a body cavity of a patient, wherein the probe comprises:

an insertion tube; and
a distal assembly, which is connected distally to the insertion tube and comprises a plurality of electrodes, which are configured to contact tissue within the body cavity;

applying radio-frequency (RF) signals simultaneously in parallel to the plurality of electrodes with an energy sufficient to ablate the tissue contacted by the electrodes; and
measuring time-varying voltage differences between the electrodes and adjusting the RF signals applied to the electrodes responsively to the measured time-varying voltage differences.

2. The method according to aspect 1, wherein adjusting the RF signals comprises regulating the RF signals so that the voltage differences do not exceed a predetermined threshold at any time during application of the RF signals.

3. The method according to aspect 2, wherein regulating the RF signals comprises adjusting an amplitude of the RF signals so as to compensate for a difference in respective peak voltages measured at a given pair of the electrodes.

4. The method according to aspect 2, wherein regulating the RF signals comprises adjusting a phase of the RF signals so as to compensate for a phase offset between respective voltage waveforms measured at a given pair of the electrodes.

5. The method according to aspect 1, wherein the distal assembly comprises a balloon, which is connected distally to the insertion tube, and is inflated within the body cavity with a fluid that flows into the balloon through the insertion tube.

6. The method according to aspect 1, and comprising fixing a common electrode to a location on the body of the patient, so that the RF signals pass through the body from the plurality of the electrodes on the probe to the common electrode, thereby ablating the tissue in a unipolar mode.

7. The method according to aspect 6, wherein adjusting the RF signals comprises controlling a proportionality between a unipolar RF current flowing from the electrodes on the probe to the common electrode and a bipolar RF current flowing between two or more of the electrodes on the probe, thereby ablating the tissue simultaneously in the unipolar mode and in a bipolar mode.

8. The method according to aspect 7, wherein controlling the proportionality between the unipolar and bipolar RF currents comprises adjusting a phase offset between respective voltage waveforms applied to the two or more of the electrodes on the probe.

**Claims**

1. A medical apparatus, comprising:

   a probe, which comprises:

   an insertion tube configured for insertion into a body cavity of a patient; and
   a distal assembly, which is connected distally to the insertion tube and comprises a plurality of electrodes, which are configured to contact tissue within the body cavity;

   an electrical signal generator, which is configured to apply radio frequency (RF) signals simultaneously to the plurality of electrodes with energy sufficient to ablate the tissue contacted by the electrodes; and
   a controller, which is coupled to the electrical signal generator, to measure time-varying voltage differences between the electrodes and to adjust the RF signals applied by the generator to the electrodes responsively to the measured time-varying voltage differences.

2. The apparatus according to claim 1, wherein the controller is configured to adjust the RF signals applied by the generator to the electrodes so that the voltage differences do not exceed a predetermined threshold at any time during application of the RF signals.

3. The apparatus according to claim 2, wherein the controller is configured to adjust an amplitude of the RF signals so as to compensate for a difference in respective peak voltages measured at a given pair of the electrodes.

4. The apparatus according to claim 2, wherein the controller is configured to adjust a phase of the RF signals so as to compensate for a phase offset between respective voltage waveforms measured at a given pair of the electrodes.

5. The apparatus according to claim 1, wherein the distal assembly comprises a balloon, which is connected distally to the insertion tube and is configured to be inflated within the body cavity with a fluid that flows into the balloon through the insertion tube.

6. The apparatus according to claim 1, and comprising a common electrode configured to be fixed to a location on the body of the patient, so that the RF signals pass through the body from the plurality of the electrodes on the probe to the common electrode, thereby ablating the tissue in a unipolar mode.

7. The apparatus according to claim 6, wherein the controller is configured to adjust the RF signals applied to the electrodes so as to control a proportionality between a unipolar RF current flowing from the electrodes on the probe to the common electrode and a bipolar RF current flowing between two or more of the electrodes on the probe, thereby ablating the tissue simultaneously in the unipolar mode and in a bipolar mode.

8. The apparatus according to claim 7, wherein the controller is configured to control the proportionality between the unipolar and bipolar RF currents by adjusting a phase offset between respective voltage waveforms applied to the two or more of the electrodes on the probe.

9. A medical apparatus, comprising:

   a probe, which comprises:

   an insertion tube configured for insertion into a body cavity of a patient; and
   a distal assembly, which is connected distally to the insertion tube and comprises a plurality of electrodes, which are configured to contact tissue within the body cavity;

   an electrical signal generator, which is configured to apply radio frequency (RF) signals simultaneously to the plurality of ablation electrodes with energy sufficient to ablate the tissue contacted by the ablation electrodes; and
   a controller coupled to the signal generator and configured to (1) determine

(a) an amplitude difference between at least two ablation electrodes of the plurality of ablation electrodes or (b) a phase difference between the at least two ablation electrodes,

and (2) adjust the amplitudes or phases to one ablation electrode of the at least two ablation electrodes or all ablation electrodes so that the amplitude difference or phase difference of the at least two ablation electrodes return to respective values that are less than predetermined respective amplitude and phase thresholds.

10. The medical apparatus of claim 9, in which the distal assembly comprises a balloon having an outer diameter of less than 15 mm and wherein the plurality of ablation electrodes are disposed radially around a longitudinal axis of the balloon.

11. The medical apparatus of claim 9, further comprising an external electrode in contact with the patient in a unipolar ablation mode.

FIG. 1

*FIG. 2*

Europäisches
Patentamt

European
Patent Office

Office européen
des brevets

# EUROPEAN SEARCH REPORT

**Application Number**

EP 21 21 7671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/272655 A1 (CONDIE CATHERINE R [US] ET AL) 1 October 2015 (2015-10-01) | 1-4,6-9, 11 | INV. A61B18/12 A61B18/14 |
| Y | * paragraphs [0003], [0009] – [0010], [0022] – [0023]; figure 1 * ----- | 5,10 | ADD. |
| Y | US 2020/205876 A1 (GOVARI ASSAF [IL]) 2 July 2020 (2020-07-02) * paragraphs [0005], [0031] * ----- | 5,10 | A61B18/00 |

**TECHNICAL FIELDS SEARCHED (IPC)**

A61B

The present search report has been drawn up for all claims

| Place of search | Date of completion of the search | Examiner |
|---|---|---|
| Munich | 12 May 2022 | Aronsson, Fredrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 21 21 7671

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2022

| Patent document cited in search report | Publication date | Patent family member(s) | Publication date |
|---|---|---|---|
| US 2015272655 A1 | 01-10-2015 | US 2015272655 A1<br>WO 2015148105 A1 | 01-10-2015<br>01-10-2015 |
| US 2020205876 A1 | 02-07-2020 | CN 111374752 A<br>EP 3682832 A1<br>JP 2020108775 A<br>US 2020205876 A1 | 07-07-2020<br>22-07-2020<br>16-07-2020<br>02-07-2020 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6584345 B
- US 10182742 B
- US 20180042674
- US 8456182 B